# EUROPEAN PATENT SPECIFICATION

(11) **EP 2 942 048 B1**
(45) Date of publication and mention of the grant of the patent: **23.08.2017**
(21) Application number: 15161428.6
(22) Date of filing: 27.03.2015
(51) Int. Cl.: A61K 8/25, A61K 8/29, A61K 8/73, A61Q 19/02, A61K 8/97, A61K 8/02

(54) **TOPICAL COMPOSITIONS COMPRISING A RESORCINOL AND POWDERS**
TOPISCHE ZUSAMMENSETZUNGEN MIT RESORCINOL UND PULVERN
COMPOSITIONS TOPIQUES COMPRENANT UN RÉSORCINOL ET DES POUDRES

(30) Priority: 27.03.2014 US 201414227260
(43) Date of publication of application: 11.11.2015
(73) Proprietor: Johnson & Johnson Consumer Inc., Skillman, NJ 08558 (US)
(72) Inventor: BRILLOUET, Anne-Sophie, Pennington, NJ New Jersey 08534 (US); DUFORT, Marisa DeVita, Hillsborough, NJ New Jersey 08844 (US); YUAN, Xudong, Morganville, NJ New Jersey 07751 (US)
(74) Representative: Carpmaels & Ransford LLP

(56) References cited:
- EP-B1- 1 299 069
- WO-A1-03/080009
- DE-A1- 10 157 490
- US-A1- 2013 071 453

## Description

### FIELD OF THE INVENTION

The present invention provides topical compositions comprising a substituted resorcinol, such as hexylresorcinol, and a solid powder having a particle size distribution width of at least 15 microns. The compositions provide the aesthetics of powder-containing compositions with the activity of resorcinols.

### BACKGROUND OF THE INVENTION

Powders are an integral part of anti-aging compositions to deliver the benefits of immediate optical modification and wrinkle blurring. Powders are also commonly used to modify the slip, viscosity, or other aesthetic features of topical compositions.

Substituted resorcinols such as 4-hexyl resorcinol are known to provide anti-aging and lightening benefits to the skin when applied topically. Examples of such resorcinols can be found in US 8318217, US 8084504, US2012/0128605, US20120128613, US2011/0081431 and US2011/0081430. Unfortunately however, 4-hexyl resorcinol can lose efficacy or activity when combined with many powders used in topical compositions.

Applicants have surprisingly discovered that the activity of substituted resorcinols can be advantageously maintained when they are used in conjunction with powders possessing a certain particle size distribution.

### SUMMARY OF THE INVENTION

The invention provides a topical composition comprising a substituted resorcinol and a solid powder having a particle size distribution width of at least 15 microns.

The invention also provides methods of treating skin by topically applying this composition to the skin.

### DETAILED DESCRIPTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

The term "comprising" encompasses "including" as well as "consisting" and "consisting essentially of" e.g. a composition "comprising" X may consist exclusively of X or may include something additional e.g. X + Y.

As used herein, "topically applying" means directly laying on or spreading on outer skin, the scalp, or hair, e.g., by use of the hands or an applicator such as a wipe, roller, or spray.

As used herein, "cosmetically acceptable" means that the ingredients the term describes are suitable for use in contact with tissues (e.g., the skin or hair) without undue toxicity, incompatibility, instability, irritation, allergic response, or the like.

Compositions of the present invention are suitable for treating signs of skin aging. As used herein, "signs of skin aging" includes the presence of lines and wrinkles, loss of elasticity, uneven skin, and blotchiness. In a particularly preferred embodiment, a sign of skin aging is the presence of lines and wrinkles and/or loss of elasticity.

As used herein, "treating signs of skin aging" refers to mitigating, reducing, preventing, improving, or eliminating the presence or signs of skin aging described above.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, the term "lightening the skin" refers generally to lightening, brightening, whitening, and/or evening of the skin tone, skin color, and/or shade of skin, and/or to the reduction in sallowness, and/or to the lightening and/or fading of hyperpigmented marks and/or lesions including, but not limited to, pigmented spots, melanin spots, age spots, sun spots, senile lentigos, freckles, lentigos simplex, pigmented solar keratosis, seborrhoeic keratosis, melasma, acne marks, post-inflammatory hyperpigmentation, lentigines, ephelides, combinations of two or more thereof and the like. In certain embodiments, "lightening the skin" also refers to increased skin radiance, glow, translucency and/or luminescence and/or obtaining a more radiant, glowing, translucent or luminous skin tone appearance or a less yellow or sallow skin tone. In certain preferred embodiments, "lightening the skin" refers to lightening and evening the skin tone, increasing skin radiance and/or lightening age spots.

As used herein, the term "skin in need of skin lightening treatment" refers generally to skin that exhibits one or more property selected from the group consisting of: skin having a measured Individual Typology Angle (ITA) value below 41 as determined per the COLIPA GUIDELINE: GUIDELINE FOR THE COLORIMETRIC DETERMINATION OF SKIN COLOUR TYPING AND PREDICTION OF THE MINIMAL ERYTHEMAL DOSE (MED) WITHOUT UV EXPOSURE published in 2007, which is incorporated herein by reference and further described below, darkened and/or sallow skin, including skin darkened by UV, skin with uneven skin tone, or skin with one or more hyperpigmented marks and/or lesions including, but not limited to, pigmented spots, melanin spots, age spots, sun spots, senile lentigos, freckles, lentigos simplex, pigmented solar keratosis, seborrhoeic keratosis, melasma, acne marks, post-inflammatory hyperpigmentation, lentigines, ephelides, combinations of two or more thereof and the like. In the COLIPA guidelines, skin color is defined function of the ITA value as: very light skin >55; Light skin 41-55, Intermediate 28-41, and Tan skin <28. In certain preferred embodiments, "skin in need of skin lightening treatment" refers to individuals with a skin having an ITA value of less than 41, such as about 40 or less, about 35 or less, about 30 or less, or more preferably about 28 or less. In certain other preferred embodiments, the present invention is directed to compositions and methods for use on skin in need of skin lightening treatment selected from sallow and/or darkened skin. In certain other preferred embodiments, the present invention is directed to compositions and methods for use on skin in need of skin lightening treatment selected from the group consisting of age spots, freckles, marks left after acne, and combinations of two or more thereof.

The topical composition comprises one or more substituted resorcinols and one or more solid powders having a particle size distribution width of at least 15 microns.

### Substituted Resorcinols

Resorcinol is a dihydroxy phenol compound (i.e., 1,3-dihydroxybenzene), having the following structure:

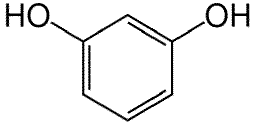

The resorcinols used herein are "substituted resorcinols." As used herein, "substituted resorcinol" means resorcinol comprising at least one substituent in the 2, 4, 5, or 6 position. Thus, the resorcinol may have as few as one and as many as four substituents. Positions 1 and 3 of the resorcinol preferably comprise an -OH group as shown above; however one or both -OH groups may be replaced by an -OR group in which R is a C₁-C₁₂ alkyl or acyl group.

Resorcinols are disclosed for example in US Patent No. 4,959,393 (Torihara, et al.), US Patent No. 6,863,897 (Love et al.) and US Published Patent Application No. 2008/0305059 (Chaudhuri et al.), the disclosures of which are incorporated herein by reference.

In certain preferred embodiments, at least one substituent on the resorcinol comprises 2 to 18 carbon atoms, preferably 2 to 12 carbon atoms, more preferably 5 to 10 carbon atoms, even more preferably 5 to 9 carbon atoms, most preferably 5 to 8 carbon atoms. In certain other embodiments, at least one substituent comprises a (linear or branched) alkyl group, such as one having the number of carbon atoms described above. Preferably, at least one substituent comprises an alkyl group that is unsaturated and linear.

In certain embodiments, the 4 position of the resorcinol is substituted, and, in certain embodiments, only the 4 position is substituted. In another embodiment, the 4 position is substituted with an akyl group. In certain preferred embodiments, the resorcinol comprises a single substituent at the 4 position that comprises an alkyl group. In certain other preferred embodiments, the resorcinol comprises a single substituent at the 4 position that consists of an alkyl group directly bonded to the benzene ring.

Particularly suitable substituted resorcinols include 4-hexyl resorcinol and 4-octylresorcinol, particularly 4-hexyl resorcinol. The structures of 4-hexylresorcinol and 4-octylresorcinol are shown below: 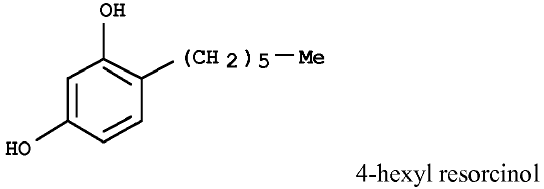 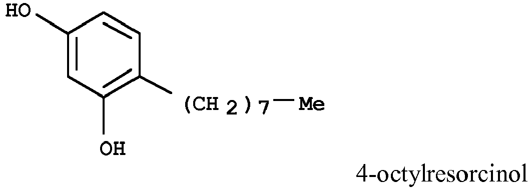 4-Hexyl resorcinol is commercially available as "SYNOVEA HR" from Sytheon of Lincoln Park, NJ. 4-Octylresorcinol is commercially available from City Chemical LLC of West Haven, Connecticut.

The substituted resorcinol is present in the composition in a safe and effective amount, such as from about 0.01% to about 10%, preferably from about 0.1% to about 5%, more preferably from about 0.1% to about 3%, more preferably from about 0.2% to about 2%, even more preferably about 1%, by weight of the composition.

### Powders

The composition also contains at least one solid powder having a particle size distribution width of at least 15 microns. In one embodiment, 80% of the powder also has particle size in the range of about 1 to about 20, or about 2 to about 25, microns.

In one embodiment, the solid powder has a particle size distribution width of at most 40 microns, preferably at most 25 microns.

Any powder suitable for use in topical or cosmetic compositions may be employed provided it is in the form of solid particles in the composition and has the recited particle size width distribution.

The powder is present in the composition in a safe and effective amount, such as up to 7%, up to 5%, or up to about 3%, by weight of the composition.

In one embodiment, the powder comprises a silicone powder.

The silicone powder may for example be a silicone resin powder, silicone elastomer powder or coated silicone elastomer powder. Coated silicone elastomer powders such as mica-coated silicone elastomer powder, sericite-coated silicone elastomer powder, talc-coated silicone elastomer powder, kaolin-coated silicone elastomer powder, boronitride-coated silicone elastomer powder, silicone resin-coated silicone elastomer powder, silicone resin-coated phenyl rubber powder, silica-coated silicone elastomer powder, titanium oxide-coated silicone elastomer powder, zinc oxide-coated silicone elastomer powder, cerium oxide-coated silicone elastomer powder, iron oxide-coated silicone elastomer powder, silica/silicone resin-coated silicone elastomer powder, titanium oxide/silicone resin-coated silicone elastomer powder, zinc oxide/silicone resin-coated silicone elastomer powder, cerium oxide/silicone resin-coated silicone elastomer powder, titanium oxide/silicone resin-coated phenyl rubber powder, zinc oxide/silicone resin-coated phenyl rubber powder, cerium oxide/silicone resin-coated phenyl rubber powder, or iron oxide/silicone resin-coated silicone elastomer powder can be used.

Preferred for use as the solid powder are silicone resin-coated silicone elastomers, such as KSP-100, KSP-101, KSP-102 and KSP-105, and silicone resin-coated phenyl rubber powder KSP-300, which are commercially available from Shin-Etsu Chemical Co., Ltd.

In another embodiment, the powder may be an absorbent powder. Water absorbent powders or oil absorbent powders may be used.

For example, an absorbent powder of plant origin that is rich in cellulose and/or silica, e.g., bamboo powder, cotton powder, wood powder, starch, starch derivatives, hydrophobically modified starches, or rice protein may be used. Boron nitride or polyacrylates may also be used.

In one embodiment, the powder is cotton powder. Cotton powder is cotton flock manufactured from textiles woven from cotton.

In another embodiment, the powder is a selected from silicas, micas and pigments.

For example, the powder may be an interference or pearl pigment. These are typically comprised of micas layered with about 50 to 300 nm films of TiO₂, Fe₂O₃, Cr₂O₃ or the like. These include white nacreous materials, such as mica covered with titanium oxide or covered with bismuth oxychloride, and colored nacreous materials, such as titanium mica with iron oxides, titanium mica with ferric blue or chromium oxide, titanium mica with an organic pigment of the aforementioned typeAlso, a mixture of silica with other ingredients, for example titanium dioxide and/or iron oxide (preferably off white iron oxide or white iron oxide), may also be used. %.

The powder may be a light diffusing powder such as ceramic microspheres made of sodium potassium aluminum silicate coated with silicone dioxide or titanium dioxide. A particularly preferred light diffusing powder of this kind is commercially available from EMD under the name RONAFLAIR LDP.

Synthetic powders may also be used. These include organic powders such as ethylene acrylate, latex, polyamide resin powder (nylon powder), cyclodextrin, polyethylene powder, methyl polymethacrylate powder, polystyrene powder, copolymer powder of styrene and acrylic acid, benzoguanamine resin powder, poly(ethylene tetrafluoride) powder, and carboxyvinyl polymer.

Other cosmetic fillers know in the cosmetic art may also be used provided they possess the required particle size distribution width.

### Topical Compositions

The compositions of the present invention are applied topically to human skin or hair. Accordingly, the composition may further include a cosmetically acceptable topical carrier that may be from about 50% to about 99.99%, by weight, of the composition (e.g., from about 80% to about 99%, by weight, of the composition). In a preferred embodiment of the invention, the cosmetically acceptable topical carrier includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition of the present invention may include water or alternatively be anhydrous or be an ointment that includes no water but organic and/or silicone solvents, oils, lipids and waxes. An ointment may contain a simple base of animal or vegetable oils or semisolid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

### Additional Cosmetically Active Agents

In one embodiment, the composition further contains another cosmetically active agent. As used herein, a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, octyl salicylate, homosalate, avobenzone, carotenoids, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides including those containing copper, coenzyme Q10, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof The cosmetically active agent will typically be present in the composition of the invention in an amount of from about 0.001% to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetylcysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

### Other Materials

Various other materials may also be present in the composition, as known in the art. These include humectants, pH adjusters, chelating agents (e.g., EDTA), fragrances, dyes, and preservatives (e.g., parabens).

The composition and formulations and products containing such compositions of the present invention may be prepared using methodology that is well known by an artisan of ordinary skill.

### Methods of Use

Compositions of the present invention may be topically applied to mammalian skin or hair, such as skin that is in need of treatment for one or more signs of skin aging as described above. In one embodiment, the compositions are applied to skin in need of treatment for lines and wrinkles and/or loss of elasticity. Compositions of the present may also be applied to skin that is in need of skin lightening treatment, for example decreased pigmentation. The compositions may be applied to the skin in need of such treatment according to a suitable treatment regimen, e.g., every month, every week, every other day, every day, twice a day, or the like.

The following non-limiting examples further illustrate the invention.

### Examples

A variety of compositions containing 4-hexyl resorcinol and different powders were made and tested for 4-hexyl resorcinol activity using pigmented epidermal equivalents in the following method.

MelanoDerm™ epidermal equivalents (commercially available from MatTek Corporation, Ashland, MA) containing melanocytes derived from a black donor were maintained in EPI-100-LLMM media according to manufacturer's protocol. The equivalents were topically treated with a test composition once daily for 5 days, in duplicate. On day 9, the tissues were evaluated for skin luminosity (L-value) using a spectrophotometer (Konica Minolta CM-2600d) & 3).

Table 1 lists powders used in the test compositions. Powders E1-E3 were according to the invention and powders C1-C5 were comparative.

**Table 1: Powders**

| Powder | Tradename | INCI | 80% Particle Size Range (10%-90%; microns) | Particle Size Distribution Width (microns) |
|---|---|---|---|---|
| C1 | SATIN PEARL 3500 | Mica; Titanium Dioxide | 2-11 | 9 |
| C2 | SEPIMAT CP5 | Methyl Methacrylate Crosspolymer | 5-15 | 10 |
| C3 | DC9701 | Dimethicone/Vinyl Dimethicone Crosspolymer; Silica | 1-10 | 9 |
| C4 | DC9506 | Dimethicone/Vinyl Dimethicone Crosspolymer | 10-15 | 14 |
| C5 | SPHERON L-1500 | Silica | 2-15 | 13 |
| E1 | KSP 101 | Vinyl Dimethicone/Methicone Silsesquioxane | 2-25 | 23 |
| E2 | RONAFLAIR LDP White | Sodium Potassium Aluminum Silicate; Titanium Dioxide; Silica | 2-20 | 18 |
| E3 | Cotton Powder | | 10-25 width, 150-500 length | 15 width, 350 length |

These powders were used in two different bases.

Table 2 lists the ingredients of Base 1. Base 1A was prepared in the same way as Base 1, except that 5% octyldodecyl neopentanoate was added.

**Table 2: Base 1**

| **INCI Name** | **% weight** |
|---|---|
| Water | Qs |
| Disodium EDTA | 0.1 |
| Ammonium Acryloyldimethyltaurate/VP Copolymer | 0.9 |
| Sclerotium Gum | 0.3 |
| Glycerin | 4.0 |
| Caprylyl Glycol | 0.5 |
| Methylparaben | 0.25 |
| Phenoxyethanol | 0.5 |
| Steareth-21 | 1.0 |
| Steareth-2 | 3.0 |
| Dimethicone; Dimethicone Crosspolymer | 2 |
| Isononyl Isononanoate | 1.5 |
| Behenyl Alcohol | 0.5 |
| Butyrospermum Parkii (Shea) Butter | 1.0 |
| Ethylhexyl Palmitate | 1.5 |
| Propylparaben | 0.15 |
| Ethylparaben | 0.15 |
| Sodium Hydroxide | 0.06 |
| Xylitylglucoside; Xylitol; Anhydroxylitol | 0.5 |

For both of the test compositions, 4-hexyl resorcinol (SYNOVEA HR) was premixed in propylene glycol at various concentrations until completely solubilized and then added into the base. The powders were post added.

Tables 3, 4, and 5 show the skin luminosity results for the different test compositions.

**Table 3**

| **Test Composition** | **Powder** | **Powder Concentration** | **L (measure of skin lightness)** |
|---|---|---|---|
| **Base 1 (placebo; no HR)** | **C1 + C2** | **0.5 + 1.5** | **39.74** |
| **Base 1 plus 0.15% HR** | **none** | **0** | **42.79** |
| **Base 1 plus 0.15% HR** | **C1 + C2** | **0.5 + 1.5** | **41.70** |
| **Base 1 plus 0.15% HR** | **C3** | **1.5** | **41.60** |
| **Base 1 plus 0.15% HR** | **C2** | **1.5** | **41.26** |
| **Base 1 plus 0.15% HR** | **C3** | **10.0** | **40.74** |
| **Base 1 plus 0.15% HR** | **C4** | **1.5** | **41.44** |
| **Base 1 plus 0.15% HR** | **C5** | **1.5** | **41.49** |
| **Base 1 plus 0.15% HR** | **E1** | **1.5** | **42.35** |
| **Base 1 plus 0.15% HR** | **E2** | **1.5** | **42.24** |
| **Base 1 plus 0.15% HR** | **E3** | **1.5** | **42.53** |

**Table 4**

| **Test Composition** | **Powder** | **Powder Concentration** | **L (measure of skin lightness)** |
|---|---|---|---|
| **Untreated control** | | | **38.61** |
| **Base 1 Placebo (no** | **C1 + C2** | **0.5 + 1.5** | **39.75** |
| **HR)** | | | |
| **Base 1 with 0.25% HR** | **none** | **0** | **44.75** |
| **Base 1 with 0.25% HR** | **C1 + C2** | **0.5 + 1.5** | **42.77** |
| **Base 1 with 0.25% HR** | **C3** | **1.5** | **43.61** |
| **Base 1 with 0.25% HR** | **C2** | **1.5** | **43.70** |
| **Base 1 with 0.25% HR** | **C3** | **10.0** | **41.78** |
| **Base 1 with 0.25% HR** | **C4** | **1.5** | **43.72** |
| **Base 1 with 0.25% HR** | **C5** | **1.5** | **43.05** |
| **Base 1 with 0.25% HR** | **E1** | **1.5** | **44.79** |
| **Base 1 with 0.25% HR** | **E2** | **1.5** | **44.36** |
| **Base 1 with 0.25% HR** | **E3** | **1.5** | **44.62** |

**Table 5**

| **Test Composition** | **Powder** | **Powder Concentration** | **L (measure of skin lightness)** |
|---|---|---|---|
| **Untreated control** | | | **40.28** |
| **Base 1A Placebo (no HR)** | **None** | **0** | **40.72** |
| **Base 1A with 0.25% HR** | **None** | **0** | **45.44** |
| **Base 1A with 0.25% HR** | **C1** | **0.5%** | **42.96** |
| **Base 1A with 0.25% HR** | **E1** | **1.5%** | **45.46** |
| **Base 1A with 0.25% HR** | **E1** | **3.0%** | **43.84** |
| **Base 1A with 0.25% HR** | **E1** | **5.0%** | **43.14** |
| **Base 1A with 0.25% HR** | **E2** | **1.5%** | **45.49** |
| **Base 1A with 0.25% HR** | **E2** | **3.0%** | **45.33** |
| **Base 1A with 0.25% HR** | **E2** | **5.0%** | **43.56** |
| **Base 1A with 0.25% HR** | **E3** | **1.5%** | **45.72** |
| **Base 1A with 0.25% HR** | **E3** | **3.0%** | **45.35** |
| **Base 1A with 0.25% HR** | **E3** | **5.0%** | **43.97** |

Treatment of the skin equivalent with test compositions containing 4-hexyl rescorinol alone advantageously increased skin luminosity, correlating with skin lightness. However, when comparative powders C1-C5 were added to these compositions, L values decreased. In contrast, when powders E1-E3 according to the invention were added to these compositions, the resulting L values were maintained.

The data in Table 5 further indicates a dose dependent effect of adding powder to hexylresorcinol-containing compositions. However, even compositions containing high levels of powders E1, E2 or E3 according to the invention (i.e., 5%) provided greater hexylresorcinol activity than a comparative composition containing only 0.5% of powder C1.
A non-exhaustive list of aspects of the invention is provided in the following numbered clauses:
1. A topical composition comprising a substituted resorcinol and a solid powder having a particle size distribution width of at least 15 microns.
2. The topical composition of clause 1, wherein the substituted resorcinol is 4-hexyl resorcinol.
3. The topical composition of clause 1, wherein the powder is a silicone elastomer.
4. The topical composition of clause 3, wherein the powder is vinyl dimethicone/methicone silsesquioxane crosspolymer.
5. The topical composition of clause 1, wherein the powder is selected from silicas, micas and pigments.
6. The topical composition of clause 5, wherein the powder comprises ceramic microspheres made of sodium potassium aluminum silicate coated with silicone dioxide or titanium dioxide.
7. The topical composition of clause 1, wherein the powder is an absorbent powder.
8. The topical composition of clause 7, wherein the powder is cotton powder.
9. The topical composition of clause 1, wherein the amount of powder is up to about 5% by weight of the composition.
10. A method of treating mammalian skin, comprising topically applying to said skin, the composition of clause 1.
11. The method of clause 10, wherein said skin is in need of improvement in a sign of skin aging.
12. The method of clause 10, wherein said skin is in need of improvement in firmness, texture, the appearance of lines or wrinkles, or the loss of elasticity.
13. The method of clause 10, wherein said skin is in need of skin lightening treatment.

## Claims

1. A topical composition comprising 4-hexyl resorcinol and a solid powder having a particle size distribution width of at least 15 microns in the 80% particle size range (10%-90%); wherein the powder is a vinyl dimethicone/methicone silsesquioxane crosspolymer.

2. The topical composition of claim 1, wherein the substituted resorcinol is present in an amount of from 0.01% to 10% by weight of the composition.

3. The topical composition of claim 1 or claim 2, wherein the amount of powder is up to about 5% by weight of the composition.

4. A cosmetic method of treating mammalian skin, comprising topically applying to said skin, the composition of any preceding claim.

5. The cosmetic method of claim 4, wherein said skin is in need of improvement in a sign of skin aging.

6. The cosmetic method of claim 4, wherein said skin is in need of improvement in firmness, texture, the appearance of lines or wrinkles, or the loss of elasticity.

7. The cosmetic method of claim 4, wherein said skin is in need of skin lightening treatment.

## Patentansprüche

1. Topische Zusammensetzung, umfassend 4-Hexylresorcinol und ein festes Pulver mit einer Teilchengrößenverteilungsbreite von mindestens 15 Mikrometern in 80%-Teilchengrößenbereich (10%-90%), wobei es sich bei dem Pulver um ein Vinyldimethicon/Methicon-Silsesquioxan-Crosspolymer handelt.

2. Topische Zusammensetzung nach Anspruch 1, wobei das substituierte Resorcinol in einer Menge von 0,01 Gew.-% bis 10 Gew.-% der Zusammensetzung vorliegt.

3. Topische Zusammensetzung nach Anspruch 1 oder Anspruch 2, wobei die Pulvermenge bis zu ungefähr 5 Gew.-% der Zusammensetzung beträgt.

4. Kosmetisches Verfahren zur Behandlung von Säuger-Haut, bei dem man die Zusammensetzung nach einem vorhergehenden Anspruch topisch auf die Haut appliziert.

5. Kosmetisches Verfahren nach Anspruch 4, wobei die Haut einer Verbesserung bezüglich eines Anzeichens von Hautalterung bedarf.

6. Kosmetisches Verfahren nach Anspruch 4, wobei die Haut einer Verbesserung in Bezug auf Festigkeit, Struktur, dem Auftreten von Falten oder Fältchen oder dem Verlust von Spannkraft bedarf.

7. Kosmetisches Verfahren nach Anspruch 4, wobei die Haut einer Hautaufhellungsbehandlung bedarf.

## Revendications

1. Composition topique comprenant du 4-hexylresorcinol et une poudre solide ayant une distribution granulométrique en largeur d'au moins 15 microns dans la plage de taille de particules de 80% (10%-90%) ; où la poudre est un polymère réticulé de vinyl diméthicone/ méthicone silsesquioxane.

2. Composition topique selon la revendication 1, dans laquelle le résorcinol substitué est présent selon une quantité allant de 0, 01% à 10% en poids de la composition.

3. Composition topique selon la revendication 1 ou la revendication 2, dans laquelle la quantité de poudre va jusqu'à environ 5% en poids de la composition.

4. Méthode cosmétique de traitement de la peau mammalienne, comprenant l'application par voie topique à ladite peau, de la composition selon l'une quelconque des revendications précédentes.

5. Méthode cosmétique selon la revendication 4, dans laquelle ladite peau a besoin d'amélioration au niveau d'un signe de vieillissement de la peau.

6. Méthode cosmétique selon la revendication 4, dans laquelle ladite peau a besoin d'amélioration au niveau de la fermeté, de la texture, de l'aspect des ridules ou des rides, ou de la perte d'élasticité.

7. Méthode cosmétique selon la revendication 4, dans laquelle ladite peau a besoin d'un traitement d'éclaircissement de la peau.
